# EUROPÄISCHE PATENTSCHRIFT

(11) **EP 1 985 248 B1**
(45) Veröffentlichungstag und Bekanntmachung des Hinweises auf die Patenterteilung: **12.08.2015**
(21) Anmeldenummer: 08103653.5
(22) Anmeldetag: 22.04.2008
(51) Int. Cl.: A61B 17/68, A61B 17/88

(54) **Schädeldeckelfixierungsvorrichtung**
Cranial flap attaching device
Dispositif de fixation de la boîte crânienne

(30) Priorität: 23.04.2007 EP 07106764
(43) Veröffentlichungstag der Anmeldung: 29.10.2008
(73) Patentinhaber: PINA Medizintechnik Vertriebs AG, 8240 Thayngen (CH); Weinacker, Marcus, 78609 Tuningen (DE)
(72) Erfinder: Weinacker, Marcus, 78609, Tuningen (DE); Matter, Georg, 8621, Wetzikon (CH)
(74) Vertreter: Graf, Werner

(56) Entgegenhaltungen:
- DE-C1- 19 832 798
- DE-U1- 29 700 269

## Beschreibung

Die Erfindung betrifft eine Schädeldeckelfixierungsvorrichtung gemäss dem Oberbegriff von Anspruch 1. Die Erfindung betrifft weiter einen Set umfassend eine Schädeldeckelfixierungsvorrichtung gemäss Anspruch 13.

### Stand der Technik

Die Druckschrift WO 2005/016160 A1 offenbart ein System zur Fixierung benachbarter Schädelknochenplatten. Die Gesamtheit der Schädelknochenplatten bildet die Schädelkalotte, im Englischen als cranial flap bezeichnet. Die Druckschrift offenbart unterschiedlich ausgestaltete Schädeldeckelklemmen, welche am Schädelknochen angeordnet werden können, wobei unterschiedliche Werkzeuge offenbart werden, um die Schädeldeckelklemme anzuziehen und dadurch an der Schädelkalotte zu befestigen.

Das offenbarte System weist die Nachteile auf, dass es schwierig ist die Schädeldeckelklemmen an der Schädelkalotte anzuordnen, und dass es zudem schwierig ist die Schädeldeckelklemme anzuziehen. Für das Anziehen der Schädeldeckelklemmen sind zudem beide Hände erforderlich. Zudem ist das Anziehen relativ zeitaufwändig.

Das Dokument DE19832798C1 offenbart ein Anlegegerät zum Fixieren einer Schädeldeckelklemme. Die Schädeldeckelklemme wird mit einem separaten Werkzeug an den Knochenplatten befestigt, daraufhin das Anlegegerät an der Schädeldeckelklemme eingefädelt, und daraufhin das Anlegegerät bedient, was zur Folge hat, dass die Schädeldeckelklemme fixiert wird. Dieses Anlegegerät ist sehr schwer und zudem sehr umständlich zu bedienen, sodass es sehr schwierig ist eine Schädeldeckelklemme auf einfache Weise an den Knochenplatten anzuordnen und schnell zu befestigen.

### Darstellung der Erfindung

Aufgabe der Erfindung ist es ein Schädeldeckelfixierungsvorrichtung zu bilden, welche es ermöglicht die Schädeldeckelklemmen auf einfache Weise anzuordnen und anzuziehen.

Diese Aufgabe wird gelöst mit einer Schädeldeckelfixierungsvorrichtung aufweisend die Merkmale von Anspruch 1. Die Unteransprüche 2 bis 12 betreffen weitere vorteilhaft ausgestaltete Schädeldeckelfixierungsvorrichtungen. Die Aufgabe wird weiter gelöst mit einem Set umfassend eine Schädeldeckelfixierungsvorrichtungen aufweisend die Merkmale von Anspruch 13.

Die erfindungsgemässe Schädeldeckelfixierungsvorrichtung weist einen handbetätigbaren Applikator auf, mit welchem die Schädeldeckelklemme verbunden ist, wobei der Applikator einen Betätigungsabschnitt aufweist, durch dessen Betätigen die Schädeldeckelklemme angezogen wird. Die Schädeldeckelklemme weist einen Stab auf, entlang welchem ein Klemmelement verschiebbar gelagert ist. Der Applikator ist derart ausgestaltet, dass ein Betätigen des Betätigungsabschnittes quer zur Verlaufsrichtung des Stabes erfolgt, vorzugsweise im Wesentlichen in zur Verlaufsrichtung des Stabes senkrechter Richtung. In einer bevorzugten Ausführungsform wird die Schädeldeckelfixierungsvorrichtung während dem Einführen der Schädeldeckelklemme in den Schädelknochen auch am Betätigungsabschnitt gehalten. Dies ergibt den Vorteil, dass die Schädeldeckelfixierungsvorrichtung mit einer einzigen Hand sowohl im Schädelknochen positioniert werden kann, und danach durch ein Drücken auf den Betätigungsabschnitt die Schädeldeckelklemme angezogen beziehungsweise fixiert werden kann. Dadurch dass der Betätigungsabschnitt quer zur Verlaufsrichtung des Stabes betätigt wird ergibt sich der Vorteil, dass die Schädeldeckelklemme sehr angenehm und präzise positionierbar und fixierbar ist, indem in einem ersten Handlungsschritt die Schädeldeckelklemme positioniert wird, und dass in einem zweiten Handlungsschritt die Schädeldeckelklemme fixiert wird, wobei währen dem Fixieren eine quer zur Stab verlaufende Kraft auf den Betätigungsabschnitt ausgeübt wird, was zur Folge hat, dass die Schädeldeckelklemme auch während dem Fixieren relativ genau in ihrer Lage gehalten werden kann, sodass das Positionieren und Fixieren der Schädeldeckelklemme unter Verwendung einer einzigen Hand durchgeführt werden kann. Die erfindungsgemässe Schädeldeckelfixierungsvorrichtung weist somit den Vorteil auf, dass diese auf eine für einen Chirurgen sehr angenehme, sichere und schnelle Weise an der Schädelkalotte fixiert werden kann. Nach dem Anziehen beziehungsweise Fixieren der Schädeldeckelklemme muss nur noch der Stab durchtrennt werden, sodass die Schädeldeckelklemme endgültig angeordnet ist und die nächste Schädeldeckelklemme gesetzt werden kann.

In einer bevorzugten Ausgestaltung ist die Schädeldeckelfixierungsvorrichtung als Einwegteil ausgestaltet, indem diese jeweils einen handbetätigbaren Applikator und eine damit verbundene Schädeldeckelklemme umfasst, wobei der handbetätigbare Applikator nach dem Positionieren und Fixieren der Schädeldeckelklemme entsorgt wird. Diese Ausgestaltung weist den Vorteil auf, dass ein Chirurge eine Mehrzahl von Schädeldeckelklemmen in kurzer Zeit nacheinander an der Schädelkalotte befestigen kann.

In einer weiteren Ausgestaltung kann der handbetätigbare Applikator auch mehrfach benutzt werden, wobei der Applikator einen Befestigungsabschnitt aufweist, an welchem jeweils eine Schädeldeckelklemme befestigbar ist, um diese in die Schädelkalotte einzuführen und mit der Schädelkalotte zu befestigen. Ein Vorteil dieser Ausgestaltung ist darin zu sehen, dass einem Chirurgen ein Set zur Verfügung gestellt werden kann, umfassend einen Applikator und eine Mehrzahl von Schädeldeckelklemmen, falls erforderlich auch unterschiedlich ausgestaltete Schädeldeckelklemmen. Ein weitere Vorteil dieser Ausgestaltung ist darin zu sehen, dass auch Schädeldeckelklemmen anderer Hersteller mit dem handbetätigbaren Applikator in die Schädelkalotte eingeführt und mit der Schädelkalotte befestigt werden können.

Nachfolgend werden mehrere Ausführungsbeispiele im Detail erläutert.

### Kurze Beschreibung der Zeichnungen

Die zur Erläuterung der Ausführungsbeispiele verwendeten Zeichnungen zeigen:
- Fig. 1: eine Seitenansicht einer Schädeldeckelklemme in geöffnetem Zustand;
- Fig. 2: eine Seitenansicht einer Schädeldeckelklemme in geschlossenem Zustand;
- Fig. 3: eine Draufsicht auf ein zweites Klemmelement der Schädeldeckelklemme;
- Fig. 4: eine Detailansicht eines Stabes der Schädeldeckelklemme;
- Fig. 5: eine Seitenansicht einer am Schädel befestigen Schädeldeckelklemme;
- Fig. 6: eine perspektivische Ansicht einer Schädeldeckelfixierungsvorrichtung, umfassend einen handbetätigbaren Applikator sowie eine Schädeldeckelklemme;
- Fig. 7: eine Ansicht der Schmalseite der in Figur 6 dargestellten Vorrichtung;
- Fig. 8: eine Seitenansicht der in Figur 6 dargestellten Vorrichtung;
- Fig. 9: eine Detailansicht eines Verbindungsteils der in Figur 6 dargestellten Vorrichtung;
- Fig. 10: ein Zustand der in Figur 6 dargestellten Vorrichtung während dem Betätigen;
- Fig. 11: ein Zustand der in Figur 6 dargestellten Vorrichtung nach dem Betätigen;
- Fig. 12: eine Seitenansicht eines weiteren Ausführungsbeispieles eines Applikators;
- Fig. 13: eine Seitenansicht eines weiteren Ausführungsbeispieles eines Applikators;
- Fig. 14: eine perspektivische Ansicht einer weiteren Schädeldeckelklemme, welche zur Verwendung mit dem Applikator geeignet ist;
- Fig. 15: eine Seitenansicht eines Endabschnittes eines Stabes;
- Fig. 16: eine perspektivische Ansicht eines weiteren Ausführungsbeispieles eines Applikators.

Grundsätzlich sind in den Zeichnungen gleiche Teile mit gleichen Bezugszeichen versehen.

### Wege zur Ausführung der Erfindung

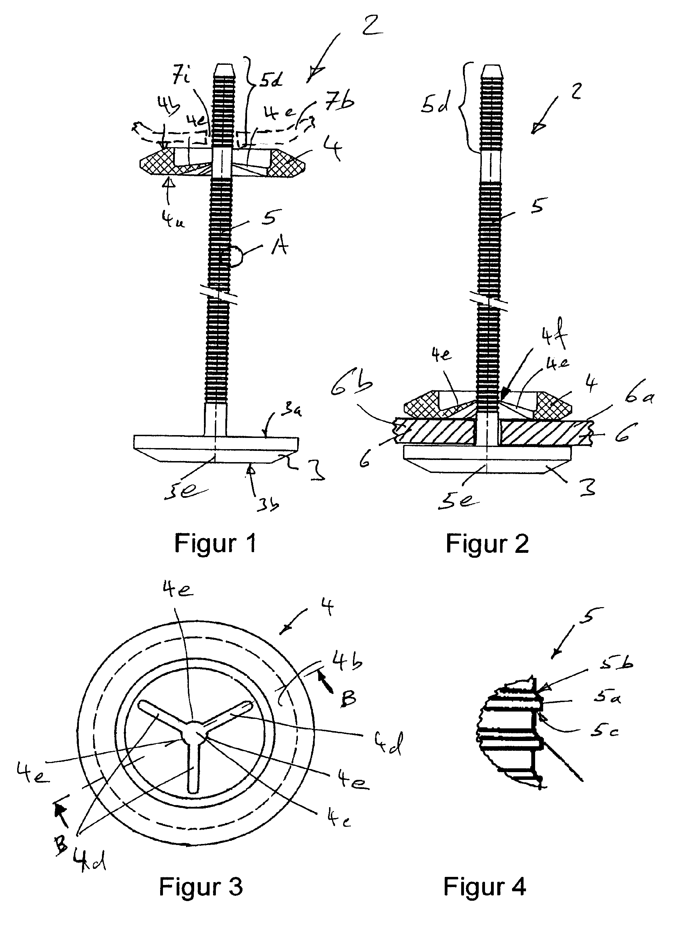

Wie in den Figuren 1 bis 5 mit einem Ausführungsbeispiel dargestellt umfasst die Schädeldeckelklemme 2 ein erstes Klemmelement 3, einen Stab 5 sowie ein zweites Klemmelement 4, welches am Stab 5 in Verlaufsrichtung des Stabes 5 verschiebbar gelagert ist. Das erste Klemmelement 3 weist eine Scheibenform mit einer ebenen Innenfläche 3a und einer konvexen Aussenfläche 3b auf. Ausgehend vom Klemmelement 3 erstreckt sich der Stab 5, welcher in einem Endabschnitt 5d endet. Der Stab 5 weist eine Längsachse 5e auf. Im dargestellten Ausführungsbeispiel ist das Klemmelement 3 und der Stab 5 als einstückiges Teil ausgestaltet. Der Stab 5 könnte jedoch auch mit dem Klemmelement 3 befestig sein, beispielsweise über ein Gewinde. Im dargestellten Ausführungsbeispiel weist der Stab 5 eine Mehrzahl von in Verlaufsrichtung des Stabes 5 beabstandet angeordneten Einrastteile 5a auf, wobei jedes Einrastteil 5a eine geneigt verlaufende Gleitstelle 5b und einen eckig ausgestaltete Einraststelle 5c aufeist. Die Ausgestaltung der Einrastteile 5a ist derart bezüglich dem zweiten Klemmelement 4 angepasst ausgestaltet, dass ein Verschieben des zweiten Klemmelementes 4, wie in den Figuren 1 und 2 dargestellt, in Richtung zum ersten Klemmelement 3 hin möglich ist, wogegen ein Verschieben in entgegen gesetzter Richtung verhindert wird. Um dies zu bewirken weist das zweite Klemmelement 4 eine Öffnung 4c sowie zur Öffnung 4c radial verlaufende Schlitze 4d auf, sodass federnde Zungen 4e ausgebildet werden, deren zur Öffnung 4c hin ausgerichtete Kante am Einrastteil 5a eingreifen. Figur 3 zeigt eine Draufsicht des zweiten Klemmelementes 4. Die Figuren 1 und 2 zeigen einen Schnitt des zweiten Klemmelementes 4 entlang der in Figur 3 dargestellten Schnittlinie B-B. Das zweite Klemmelement 4 umfasst zudem eine Innenfläche 4a und eine Aussenfläche 4b. An der Aussenfläche 4b greift, wie in Figur 1 strichliert dargestellt, ein Stossteil 7b an, um auf das zweite Klemmelement 4 eine nach unten, zum ersten Klemmelement 3 hin wirkende Kraft auszuüben, um dadurch das zweite Klemmelement 4 in die in Figur 2 dargestellte Lage zu verschieben. Das Stossteil 7b weist vorzugweise eine Bohrung 7i auf, welche den Stab 5 umschliesst, sodass das Stossteil 7b durch den Stab in Verlaufsichtung 5e des Stabes 5 geführt ist.

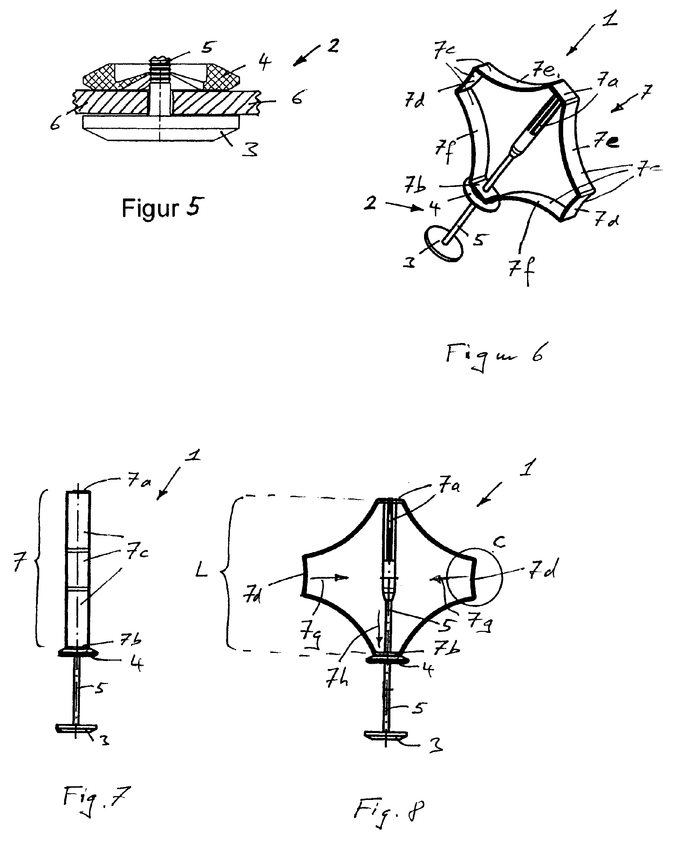

Die in Figur 1 dargestellte Schädeldeckelklemme 2 wird in einem ersten Verfahrensschritt zur Fixierung von benachbarten Schädelknochenplatten derart eingeführt, dass das erste Klemmelement 3 unter die Schädelkalotte zu liegen kommt. Nach dem Positionieren der Schädeldeckelklemme 2 wird diese angezogen beziehungsweise in eine Klemmstellung gebracht, indem, wie in Figur 2 dargestellt, das zweite Klemmelement 4 derart verschoben wird, dass dieses auf den Knochenplatten 6 aufliegt, in dargestellten Beispiel einen Knochendeckel 6a und einen Schädel 6b. Nach diesem Verfahrensschritt wird der Stab 5 durchtrennt, sodass die Schädeldeckelklemme 2 wie in Figur 5 in einem Schnitt dargestellt an der Schädelkalotte anliegt und benachbarte Schädelknochenplatten in ihrer Lage fixiert.

Die Schädeldeckelklemme 2 kann aus jedem geeigneten, biokompatiblen Material, wie etwa rostfreiem Stahl, Titan, einer Legierung auf Titanbasis, einem Kunststoff oder einem resorbierbaren Material gefertigt sein. Wenn die Schädeldeckelklemme 2 aus einem metallischen Material gefertigt ist, so sind das erste und zweite Klemmelement 3,4 sowie der Stab 5 vorzugsweise aus demselben Material gefertigt. Als besonders gut geeignet ist ein Material aus PEEK oder PEKK, und insbesondere auch das Material OXPEKK^{®}. Diese Materialien weisen auch den Vorteil auf, dass diese bei klinischen Untersuchungen wie Röntgen, CT oder MRT keine Artefakte bewirken.

Figur 6 zeigt in einer perspektivischen Ansicht eine Schädeldeckelfixierungsvorrichtung 1 umfassend eine Schädeldeckelklemme 2, welche am Verbindungsabschnitt 5d mit einem handbetätigbaren Applikator 7 verbunden ist. Der handbetätigbare Applikator 7 umfasst ein Halteteil 7a und ein Stossteil 7b, welche über zwei Verbindungsteile 7c miteinander verbunden sind. Die beiden Verbindungsteile 7c sind bezüglich dem Stab 5 beziehungsweise bezüglich der Verlaufsachse 5e des Stabes 5 gegenseitig symmetrisch verlaufend angeordnet. Das Stossteil 7b weist, wie in Figur 1 im Detail dargestellte, eine Bohrung 7i auf, sodass das Stossteil 7b am Stab 5 geführt ist. Das Stossteil 7b ist mit beiden Verbindungsteilen 7c fest verbunden. Das Verbindungsteil 7c könnte jedoch auch derart ausgestaltet sein, dass dieses ein zum Stossteil 7b hin angeordnetes Auflageteil aufweist, welches nur auf dem Stossteil 7b aufliegt, aber mit diesem nicht fest verbunden ist.

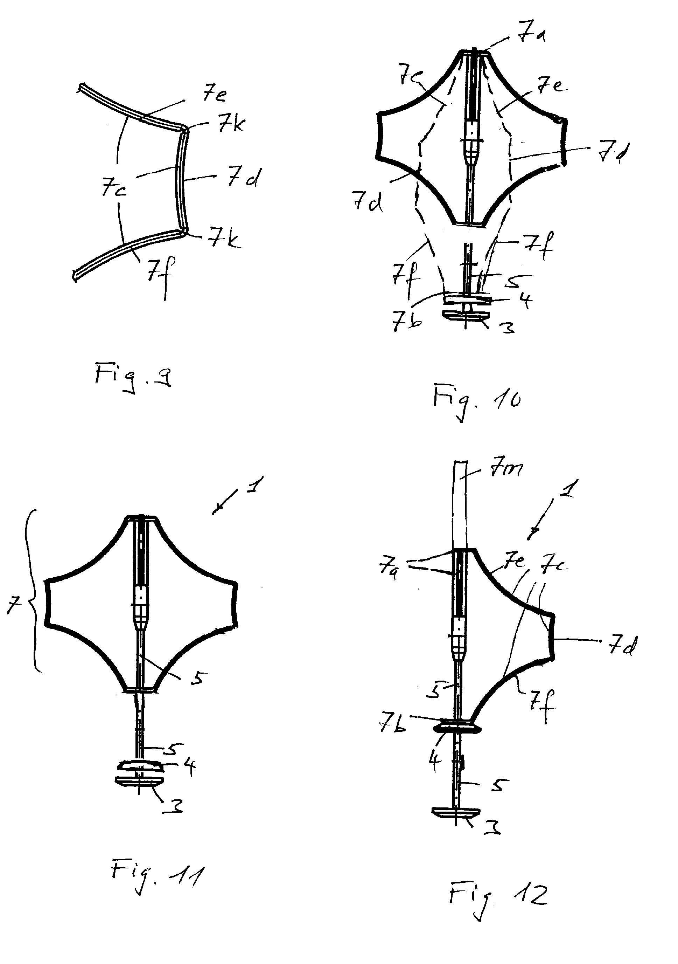

Das Verbindungsteil 7c besteht nacheinander folgend aus einem ersten Verbindungsabschnitt 7e, einem Betätigungsabschnitt 7d und einem zweiten Verbindungsabschnitt 7f. Vorzugsweise verläuft der Betätigungsabschnitt 7d im Wesentlichen parallel zum Stab 5. Vorteilhafterweise verläuft zumindest einer der ersten und zweiten Verbindungsabschnitte 7e, 7f gekrümmt, besonders vorteilhaft wie in Figur 6 dargestellt konkav bezüglich dem Stab 5. Figur 7 zeigt die Schmalseite der in Figur 6 dargestellten Vorrichtung 1. Figur 8 zeigt eine Seitenansicht der in Figur 6 dargestellten Vorrichtung 1. Der handbetätigbare Applikator 7 ist derart ausgestaltet, dass ein Verschieben der beiden Betätigungsabschnitte 7d in Bewegungsrichtung 7g, das heisst zum Stab 5 hin, zur Folge hat, dass sich das Stossteil 7b in Richtung zum ersten Klemmelement 3 hin bewegt, was zur Folge hat, dass das zweite Klemmelement 4 durch das Stossteil 7b in Richtung zum ersten Klemmelement 3 hin verschoben wird. Vorzugsweise liegt am einen Betätigungsabschnitt 7d der Daumen an, wogegen am zweiten Betätigungsabschnitt der Zeigefinger anliegt. Dies ermöglichst einerseits das erste Klemmelement 3 in der in Figur 1 dargestellten Lage in die Schädelkalotte einzuführen. Danach werden die beiden Finger gegeneinander gepresst, sodass die Betätigungsabschnitte 7d zueinander hin bewegt werden, wodurch das zweite Klemmelement 4 wie in Figur 2 dargestellt verschoben werden kann, bis das zweite Klemmelement beispielsweise auf der Schädelkalotte 6 aufliegt. Figur 10 zeigt in einer Seitenansicht die Schädeldeckelfixierungsvorrichtung 1 in dieser Lage. Figur 11 zeigt in einer Seitenansicht die Schädeldeckelfixierungsvorrichtung 1 nachdem die auf die Betätigungsabschnitte 7 wirkende Kraft wieder reduziert wurde, sodass das Stossteil 7b wieder die Ausgangslage einnimmt, wogegen das zweite Klemmelement 4 in der in Figur 2 dargestellten Lage verharrt.

Währen dem Einführen des ersten Klemmelementes 3 in die Schädelkalotte ist es von Vorteil, wenn der Abstand zwischen dem ersten und zweiten Klemmelement 3,4 genügen gross ist, um während dem Einführung dem Chirurgen eine möglichst grosse Bewegungsfreiheit zu ermöglichen. Damit der handbetätigbare Applikator 7 beziehungsweise das Stossteil 7b einen relativ grossen Verschiebungsweg aufweist ist es von Vorteil, wenn der Abstand zwischen dem Betätigungsabschnitt 7d und dem Stab 5 zumindest ein Viertel der sich in Verlaufsrichtung des Stabes 5 erstreckenden Länge L des Verbindungsteils 7c beträgt. Vorzugsweise beträgt dieser Abstand, wie in Figur 8 dargestellt, zumindest die Hälfte.

Die Schädeldeckelfixierungsvorrichtung 1 ist vorzugsweise als Einwegteil ausgestaltet, und ist vorzugsweise aus einem Kunststoff gefertigt. Die Schädeldeckelklemme 2 kann auch lösbar mit dem Applikator 7 verbunden sein, beispielsweise indem der Verbindungsabschnitt 5d als Gewinde ausgestaltet ist, welches mit einer Bohrung des Halteteils 7a verbunden ist. Der Applikator 7 kann beispielsweise auch als mehrfach verwendbare Vorrichtung ausgestaltet sein, welche mit Schädeldeckelklemmen 2 bestückt wird.

Figur 9 zeigt den in Figur 8 mit C bezeichneten Ausschnitt im Detail. Ersichtlich ist das Verbindungsteil 7c, welches einen ersten Verbindungsabschnitt 7e, einen Betätigungsabschnitt 7d sowie einen Verbindungsabschnitt 7f aufweist, wobei die vorhin genannten Abschnitte 7e, 7d, 7f über elastische, gelenkige Stellen 7k miteinander verbunden sind. Das Verbindungsteil 7c ist vorzugsweise aus einem Kunststoff gefertigt.

Figur 12 zeigt in einer Seitenansicht eine weitere Ausführungsform einer Schädeldeckelfixierungsvorrichtung 1, welche im Unterschied zu der in Figur 8 dargestellten Ausführungsform, nur ein einziges Verbindungsteil 7c aufweist. Der Applikator 7 ist beispielsweise derart betätigbar, dass der Daumen auf dem Betätigungsabschnitt 7d aufliegt, wogegen der Zeigefinger auf dem Halteteil 7a oder dem Stab 5 aufliegt. In einem weiteren Ausführungsbeispiel kann der Applikator 7 zudem wie dargestellt noch einen Haltegriff 7m aufweisen, welcher zum besseren Führen des Applikators 7 dienen kann.

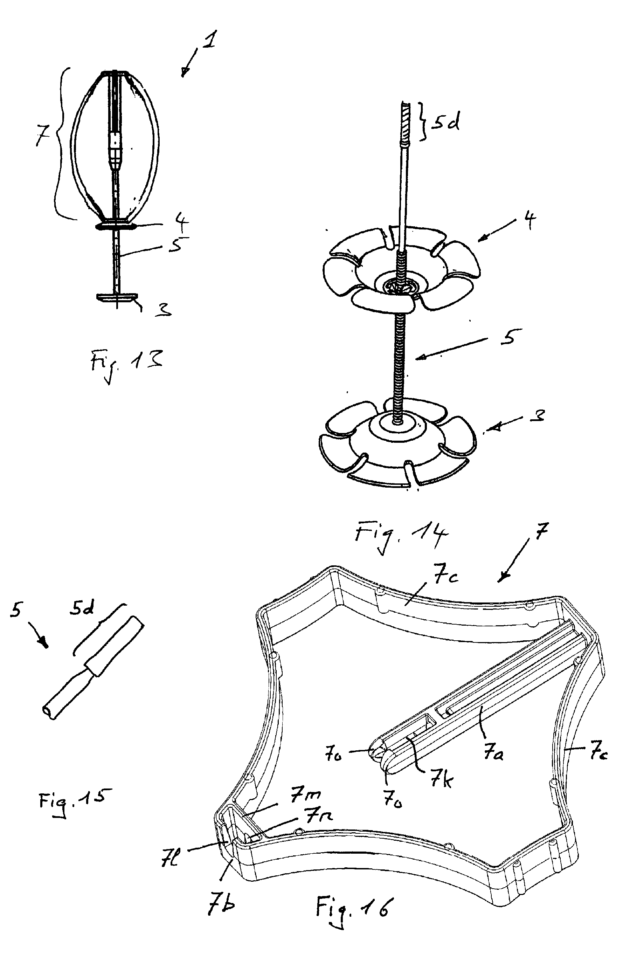

Figur 13 zeigt ein weiteres Ausführungsbeispiel eines Applikators 7, welcher zwei Verbindungsteile 7c aufweist, die beide kurvenförmig verlaufen.

Figur 14 zeigt ein weiteres Ausführungsbeispiel einer Schädeldeckelklemme 2. Der handbetätigbare Applikator 7 sowie die Schädeldeckelklemmen 2 sind vorzugsweise derart gegenseitig angepasst ausgestaltet, dass die Schädeldeckelklemme 2, beispielsweise über den Verbindungsabschnitt 5d, fest mit dem Applikator 7 verbindbar sind. Somit ist es möglich, dass der Applikator 7 mit einer Vielzahl von unterschiedlich ausgestalteten Schädeldeckelklemmen 2 verwendet werden kann, um diese an der Schädelkalotte einzuführen und zu befestigen. Der Verbindungsabschnitt 5d kann mit einer Vielzahl unterschiedlicher Methoden mit dem Applikator 7 verbunden sein, z.B. mit einer Schraub-, Steck- oder Quetschverbindung.

Figur 15 zeigt in einer Seitenansicht ein weiteres Ausführungsbeispiel eines Verbindungsabschnittes 5d eines Stabes 5. Der in Figur 16 dargestellte Applikator 7 weist ein Halteteil 7a auf, wobei das Halteteil 7a eine Ausnehmung 7k aufweist, welche derart zu dem in Figur 15 dargestellten Verbindungsabschnitt 5d angepasst ausgestaltet ist, dass der Stab 5 fest mit dem Halteteil 7a verbindbar ist. In einer vorteilhaften Ausgestaltung wird der Verbindungsabschnitt 5d seitlich, das heisst senkrecht zur Verlaufsrichtung des Stabes 5, in die in Figur 16 dargestellte Ausnehmung 7k eingeführt, sodass der Verbindungsabschnitt 5d innerhalb die Ausnehmung 7k zu liegen kommt und dadurch gehalten ist.

In einer weiteren vorteilhaften Ausgestaltung könnte der Verbindungsabschnitt 5d auch in Verlaufsrichtung des Halteteils 7a beziehungsweise in Verlaufsrichtung des Stabes 5 bewegt werden, und dabei ausgehend von den Backen 7o zwischen die beiden Backen 7o und danach in die Ausnehmung 7k eingeschoben werden, wobei die beiden Backen 7o vorzugsweise federnd oder elastisch ausgestaltet sind, sodass der Verbindungsabschnitt 5d nach dem Einschieben in die Ausnehmung 7k fest im Halteteil 7a gehalten ist. Stab 5

In einer vorteilhaften Ausgestaltung kann der Verbindungsabschnitt 5d durch eine seitliche Bewegung, das heisst eine Bewegung senkrecht zur Verlaufsrichtung des Stabes 5, wieder aus der Ausnehmung 7k entfernt werden, sodass wieder ein neuer Stab 5 im Halteteil 7a des Applikators 7 verankert werden kann. Somit kann der Applikator 7 mehrmals verwendet werden. In einer weiteren vorteilhaften Ausgestaltung weist das Stossteil 7b eine Nut 71 auf. In einer weiteren vorteilhaften Ausgestaltung ist neben dem Stossteil 7b ein weiteres Führungsteil 7m mit einer Nut 7n angeordnet. In einer besonders vorteilhaften Ausgestaltung verlaufen die beiden Nuten 71, 7n senkrecht zur Verlaufsrichtung des Stabes 5 und in derselben Richtung wie die Ausnehmung 7k. Beim Entfernen des Stabes 5 aus der Ausnehmung 7k bilden die beiden Nuten 71, 7n eine Führung des Stabes 5, sodass der Verlauf der beiden Nuten 71, 7n die dem Stab 5 mögliche Schwenkbewegung beziehungsweise die Schwenkrichtung definieren. Bei einem wieder verwendbaren Applikator 7 muss der Stab 5 nach dem Setzen der Schädeldeckelklemme 2 wieder aus dem Applikator 7 entfernt werden. Die Nuten 71, 7n erlauben es den Stab 5 durch eine definierte Schwenkbewegung aus der Ausnehmung 7k zu heben und danach aus dem Applikator 7 zu entfernen.

## Patentansprüche

1. Schädeldeckelfixierungsvorrichtung (1) umfassend eine Schädeldeckelklemme (2) sowie einen handbetätigbaren Applikator (7), wobei die Schädeldeckelklemme (2) ein erstes und ein zweites Klemmelement (3,4) sowie einen Stab (5) umfasst, wobei sich der Stab (5) vom ersten Klemmelement (3) aus erstreckt und in einem Endabschnitt (5d) endet, und wobei das zweite Klemmelement (4) in Verlaufsrichtung (5e) des Stabes (5) verschiebbar angeordnet ist, und wobei der handbetätigbare Applikator (7) ein Halteteil (7a) und ein Stossteil (7b) aufweist, wobei das Halteteil (7a) und das Stossteil (7b) über ein Verbindungsteil (7c) miteinander verbunden sind, und wobei der Endabschnitt (5d) des Stabes (5) vom Halteteil (7a) gehalten ist, und wobei das Stossteil (7b) derart angeordnet ist, dass dieses auf das zweite Klemmelement (4) eine gegen das erste Klemmelement (3) hin ausgerichtete Kraft ausüben kann, und wobei das Verbindungsteil (7c) nacheinander folgend einen ersten Verbindungsabschnitt (7e), einen Betätigungsabschnitt (7d) und einen zweiten Verbindungsabschnitt (7f) aufweist, und wobei zumindest einer der ersten und zweiten Verbindungsabschnitte (7e,7f) gekrümmt verläuft, **dadurch gekennzeichnet, dass** das Verbindungsteil (7c) derart teilweise beabstandet zum Stab (5) verläuft, dass der Betätigungsabschnitt (7d) im Wesentlichen parallel zum Stab (5) verläuft und bezüglich dem Stab (5) beabstandet verläuft, sodass ein Verschieben des Betätigungsabschnittes (7d) des Verbindungsteils (7c) zum Stab (5) hin den Abstand zwischen Halteteil (7a) und Stossteil (7b) vergrössert, um dadurch das zweite Klemmelement (4) über das darauf einwirkende Stossteil (7b) in Richtung des ersten Klemmelementes (3) zu verschieben, und dass der handbetätigbare Applikator (7) aus einem einzigen Teil besteht.

2. Vorrichtung nach Anspruch 1, **dadurch gekennzeichnet, dass** der erste Verbindungsabschnitt (7e), der Betätigungsabschnitt (7d) sowie der zweite Verbindungsabschnitt (7f) über elastische, gelenkige Stellen (7k) miteinander verbunden sind.

3. Vorrichtung nach Anspruch 2, **dadurch gekennzeichnet, dass** das Verbindungsteil (7c) aus einem Kunststoff gefertigt ist.

4. Vorrichtung nach einem der vorhergehenden Ansprüche, **dadurch gekennzeichnet, dass** der Applikator (7) zwei Verbindungsteile (7c) aufweist, welche gegenseitig symmetrisch verlaufend angeordnet sind.

5. Vorrichtung nach einem der vorhergehenden Ansprüche, **dadurch gekennzeichnet, dass** das Stossteil (7b) eine Bohrung (7i) aufweist, durch welche der Stab (5) verläuft.

6. Vorrichtung nach einem der vorhergehenden Ansprüche, **dadurch gekennzeichnet, dass** das Stossteil (7b) mit dem Verbindungsteil (7c) fest verbunden ist.

7. Vorrichtung nach einem der vorhergehenden Ansprüche, **dadurch gekennzeichnet, dass** zumindest einer der ersten und zweiten Verbindungsabschnitte (7e,7f) bezüglich dem Stab (5) konkav verläuft.

8. Vorrichtung nach einem der vorhergehenden Ansprüche, **dadurch gekennzeichnet, dass** der Abstand zwischen dem Betätigungsabschnitt (7d) und dem Stab (5) zumindest ein Viertel der sich in Verlaufsrichtung des Stabes (5) erstreckenden Länge des Verbindungsteils (7c) beträgt, und vorzugsweise zumindest die Hälfte.

9. Vorrichtung nach einem der vorhergehenden Ansprüche, **dadurch gekennzeichnet, dass** diese als Einwegteil ausgestaltet ist.

10. Vorrichtung nach einem der Ansprüche 1 bis 8, **dadurch gekennzeichnet, dass** die Schädeldeckelklemme (2) lösbar mit dem Applikator (7) verbunden ist.

11. Vorrichtung nach einem der vorhergehenden Ansprüche, **dadurch gekennzeichnet, dass** das zweite Klemmelement (4) Zungen (4e) aufweist, dass der Stab (5) eine Mehrzahl von Einraststellen (5c) aufweist, und dass die Zungen (4e) und die Einraststellen (5c) derart zusammenwirken, dass das zweite Klemmelement (4) gehindert ist, eine Bewegung in Richtung zum Verbindungsabschnitt (5d) hin auszuführen.

12. Vorrichtung nach einem der vorhergehenden Ansprüche, **dadurch gekennzeichnet, dass** das Halteteil (7a) einen Handgriff umfasst.

13. Set umfassend eine Schädeldeckelfixierungsvorrichtung (1) nach einem der vorhergehenden Ansprüche, mit einem handbetätigbaren Applikator (7) sowie eine Mehrzahl von Schädeldeckelklemmen (2), welche mit dem Halteteil (7a) des Applikators (7) verbindbar sind.

## Claims

1. Cranium fixing device (1) comprising a cranium clamp (2) and also a manually operable applicator (7), wherein the cranium clamp (2) comprises a first and a second clamping element (3, 4) and also a rod (5), wherein the rod (5) extends from the first clamping element (3) and ends in an end portion (5d), and wherein the second clamping element (4) is arranged displaceably in a direction of extent (5e) of the rod (5), and wherein the manually operable applicator (7) has a holding part (7a) and a thrust part (7b), wherein the holding part (7a) and the thrust part (7b) are connected to one another via a connecting part (7c), and wherein the end portion (5d) of the rod (5) is held by the holding part (7a), and wherein the thrust part (7b) is arranged in such a manner that it can exert a force on the second clamping element (4) which is oriented towards the first clamping element (3), and wherein the connecting part (7c) has, in succession, a first connecting portion (7e), an actuating portion (7d) and a second connecting portion (7f), and wherein at least one of the first and second connecting portions (7e, 7f) has a curved extent, **characterized in that** the connecting part (7c) extends in part spaced apart from the rod (5) in such a manner that the actuating portion (7d) extends substantially parallel to the rod (5) and extends spaced apart with respect to the rod (5), such that displacement of the actuating portion (7d) of the connecting part (7c) towards the rod (5) increases the distance between the holding part (7a) and the thrust part (7b), in order to thereby displace the second clamping element (4) via the thrust part (7b) acting thereon in the direction of the first clamping element (3), and **in that** the manually operable applicator (7) consists of a single part.

2. Device according to Claim 1, **characterized in that** the first connecting portion (7e), the actuating portion (7d) and also the second connecting portion (7f) are connected to one another via elastic hinged points (7k).

3. Device according to Claim 2, **characterized in that** the connecting part (7c) is produced from a plastic.

4. Device according to one of the preceding claims, **characterized in that** the applicator (7) has two connecting parts (7c) arranged so as to extend in a mutually symmetrical manner.

5. Device according to one of the preceding claims, **characterized in that** the thrust part (7b) has a bore (7i), through which the rod (5) extends.

6. Device according to one of the preceding claims, **characterized in that** the thrust part (7b) is fixedly connected to the connecting part (7c).

7. Device according to one of the preceding claims, **characterized in that** at least one of the first and second connecting portions (7e, 7f) extends concavely with respect to the rod (5).

8. Device according to one of the preceding claims, **characterized in that** the distance between the actuating portion (7d) and the rod (5) amounts to at least a quarter, and preferably at least half, of the length of the connecting part (7c) extending in the direction of extent of the rod (5).

9. Device according to one of the preceding claims, **characterized in that** said device is configured as a disposable part.

10. Device according to one of Claims 1 to 8, **characterized in that** the cranium clamp (2) is connected detachably to the applicator (7).

11. Device according to one of the preceding claims, **characterized in that** the second clamping element (4) has tongues (4e), **in that** the rod (5) has a plurality of latch-in points (5c), and **in that** the tongues (4e) and the latch-in points (5c) interact in such a manner that the second clamping element (4) is prevented from executing a movement in the direction towards the connecting portion (5d).

12. Device according to one of the preceding claims, **characterized in that** the holding part (7a) comprises a handle.

13. Set comprising a cranium fixing device (1) according to one of the preceding claims, having a manually operable applicator (7) and also a plurality of cranium clamps (2), which can be connected to the holding part (7a) of the applicator (7).

## Revendications

1. Dispositif de fixation de calotte crânienne (1) comprenant une pince de calotte crânienne (2) ainsi qu'un applicateur actionnable manuellement (7), dans lequel la pince de calotte crânienne (2) comprend un premier et un deuxième éléments de serrage (3, 4) ainsi qu'une tige (5), dans lequel la tige (5) s'étend à partir du premier élément de serrage (3) et se termine par une section d'extrémité (5d), et dans lequel le deuxième élément de serrage (4) est disposé de façon déplaçable dans la direction d'extension (5e) de la tige (5), et dans lequel l'applicateur actionnable manuellement (7) présente une partie de maintien (7a) et une partie de poussée (7b), dans lequel la partie de maintien (7a) et la partie de poussée (7b) sont reliées l'une à l'autre par une partie de liaison (7c), et dans lequel la section d'extrémité (5d) de la tige (5) est maintenue par la partie de maintien (7a), et dans lequel la partie de poussée (7b) est disposée de telle manière que celle-ci puisse exercer sur le deuxième élément de serrage (4) une force dirigée vers le premier élément de serrage (3), et dans lequel la partie de liaison (7c) présente à la suite l'une de l'autre une première section de liaison (7e), une section d'actionnement (7d) et une deuxième section de liaison (7f), et dans lequel au moins une de la première et de la deuxième sections de liaison (7e, 7f) présente une forme courbe, **caractérisé en ce que** la partie de liaison (7c) s'étend en partie à distance de la tige (5), de telle manière que la section d'actionnement (7d) soit essentiellement parallèle à la tige (5), de telle manière qu'un déplacement de la section d'actionnement (7d) de la partie de liaison (7c) vers la tige (5) augmente la distance entre la partie de maintien (7a) et la partie de poussée (7b), afin de déplacer ainsi le deuxième élément de serrage (4) au moyen de la partie de poussée (7b) agissant sur celui-ci en direction du premier élément de serrage (3), et **en ce que** l'applicateur actionnable manuellement (7) est composé d'une seule pièce.

2. Dispositif selon la revendication 1, **caractérisé en ce que** la première section de liaison (7e), la section d'actionnement (7d) ainsi que la deuxième section de liaison (7f) sont reliées l'une à l'autre au moyen de points articulés élastiques (7k).

3. Dispositif selon la revendication 2, **caractérisé en ce que** la partie de liaison (7c) est fabriquée en une matière plastique.

4. Dispositif selon l'une quelconque des revendications précédentes, **caractérisé en ce que** l'applicateur (7) présente deux parties de liaison (7c), qui sont disposées symétriquement l'une par rapport à l'autre.

5. Dispositif selon l'une quelconque des revendications précédentes, **caractérisé en ce que** la partie de poussée (7b) présente un alésage (7i), à travers lequel passe la tige (5).

6. Dispositif selon l'une quelconque des revendications précédentes, **caractérisé en ce que** la partie de poussée (7b) est fixement reliée à la partie de liaison (7c).

7. Dispositif selon l'une quelconque des revendications précédentes, **caractérisé en ce qu'**au moins une des premières et deuxièmes sections de liaison (7e, 7f) est de forme concave par rapport à la tige (5).

8. Dispositif selon l'une quelconque des revendications précédentes, **caractérisé en ce que** la distance entre la section d'actionnement (7d) et la tige (5) vaut au moins un quart de la longueur de la partie de liaison (7c) s'étendant dans la direction d'extension de la tige (5), et de préférence au moins la moitié.

9. Dispositif selon l'une quelconque des revendications précédentes, **caractérisé en ce que** celui-ci est réalisé comme pièce à usage unique.

10. Dispositif selon l'une quelconque des revendications 1 à 8, **caractérisé en ce que** la pince de calotte crânienne (2) est reliée de façon séparable à l'applicateur (7).

11. Dispositif selon l'une quelconque des revendications précédentes, **caractérisé en ce que** le deuxième élément de serrage (4) présente des languettes (4e), **en ce que** la tige (5) présente une multiplicité de points d'encliquetage (5c), et **en ce que** les languettes (4e) et les points d'encliquetage (5c) coopèrent de telle manière que le deuxième élément de serrage (4) soit empêché d'exécuter un mouvement en direction de la section de liaison (5d).

12. Dispositif selon l'une quelconque des revendications précédentes, **caractérisé en ce que** la partie de maintien (7a) comprend une poignée.

13. Ensemble comprenant un dispositif de fixation de calotte crânienne (1) selon l'une quelconque des revendications précédentes, avec un applicateur actionnable manuellement (7) ainsi qu'une multiplicité de pinces de calotte crânienne (2), qui peuvent être reliées à la partie de maintien (7a) de l'applicateur (7).
